Europäisches Patentamt

European Patent Office

Office européen des brevets

(11) Veröffentlichungsnummer : **0 016 906**
**B1**

(12) EUROPÄISCHE PATENTSCHRIFT

(45) Veröffentlichungstag der Patentschrift :
17.10.84

(51) Int. Cl.³ : **A 61 F   1/00, A 61 C   8/00**

(21) Anmeldenummer : 80100293.2

(22) Anmeldetag : 22.01.80

(54) Knochenzement und Verfahren zu seiner Herstellung.

(30) Priorität : 16.02.79 DE 2905878

(43) Veröffentlichungstag der Anmeldung :
15.10.80 Patentblatt 80/21

(45) Bekanntmachung des Hinweises auf die Patenterteilung : 17.10.84 Patentblatt 84/42

(84) Benannte Vertragsstaaten :
AT BE CH DE FR GB IT NL SE

(56) Entgegenhaltungen :
DE-A- 2 501 683
DE-A- 2 518 153
DE-A- 2 614 170
DE-A- 2 620 907
DE-A- 2 724 814
DE-A- 2 733 394
DE-A- 2 742 128
DE-A- 2 821 354
US-A- 4 141 864
J. Biomed. Nater. Res., Vol. 11, 1977, John Wiley &
Sons, Inc. A. N. Rijke et al. "Porous Acrylic Cement",
Seiten 373-394
Die Akte enthält technische Angaben, die nach dem
Eingang der Anmeldung eingereicht wurden und die
nicht in dieser Patentschrift enthalten sind.

(73) Patentinhaber : Merck Patent Gesellschaft mit beschränkter Haftung
Frankfurter Strasse 250
D-6100 Darmstadt (DE)

(72) Erfinder : Draenert, Klaus, Dr.
Uhlandstrasse 16
D-8012 Ottobrunn (DE)

**Beschreibung**

Die Erfindung betrifft einen Knochenzement auf der Basis von polymeren Acrylaten, insbesondere Polymethylmethacrylat, der insbesondere als Knochenersatz-, Knochenverbund- und Prothesenverankerungsmaterial Verwendung finden kann. Die Erfindung betrifft ferner ein Verfahren zur Herstellung eines derartigen Knochenzementes.

Die in den Körper implantierten Knochenzemente werden vom Körper nicht resorbiert, sondern nach dem Einwachsen von körpereigenem Gewebe umschlossen. Eine stabile Verbindung Implantat/Körper ist nur dann gegeben, wenn das Implantat « knöchern » einwächst, das heißt also, daß Knochengewebe bis unmittelbar an die Implantatoberfläche reicht. Sehr häufig kommt es jedoch durch biomechanische Überbeanspruchung des Implantats, d. h. auf das Implantat wirkende Biege- und Scherkräfte, die von der relativ kleinen Grenzfläche Implantat/Körper aufgefangen werden müssen, zur Ausbildung von Umbauzonen mit Geflechtknochen und schließlich zur Bildung einer straffen Bindegewebsschicht anstelle von Knochengewebe. Dies führt zu einer Lockerung des Implantats.

Es hat nun nicht an Versuchen gefehlt, diesen Zustand dadurch zu verbessern, daß die Grenzfläche Implantat/Körper vergrößert wird, z. B. dadurch, daß das Implantat mit einer Porenstruktur versehen wird, die es gestattet, daß das Implantat vollständig von Körpergewebe durchwachsen wird. So wird in der DE-A-2 008 708 insbesondere für Zahnimplante vorgeschlagen, dem Acrylpolymeren neben geriebener Wurzelsubstanz von Naturzähnen und gegebenenfalls einem Schäummittel bis zu 30 Gew.% geriebenen « anorganischen Knochen » zuzusetzen. Durch Auflösung des anorganischen Knochens im Körper soll eine hohe Porosität ausgebildet werden, die su einem festen Verbund mit dem angrenzenden Gewebe führen soll.

In der DE-A-2 620 890 wird vorgeschlagen, dem Polymeren Calciumphosphat in einem Mengenverhältnis von 1 : 1 bis 5 : 1 zuzusetzen, was einem Calciumphosphatanteil von 50 bis 83 % entspricht. Auch Calciumphosphat wird im Körper abgebaut und resorbiert, so daß auch hier eine sehr hohe Porosität entsteht, die durch Nachwachsen von Körpergewebe zu einem haltbaren Verbund Implantat/Körper führen soll. Das gleiche Prinzip ist auch bei der in der DE-A-2 620 907 beschriebenen Prothesenverankerung angewendet, wo Calciumphosphatpartikel von 0,5 bis 1 mm Durchmesser so als dichte Kugelschüttung aufgebracht sind, daß nach der Resorption das Calciumphosphat ein durchgehendes Porensystem entstehen soll, das durch Körpergewebe ausgefüllt werden kann.

Auch bei dem in der DE-A-2 518 153 beschriebenen Verfahren soll durch Zumischung eines wäßrigen Gels zu der Polymerenmischung ein Netzwerk aus Gelfäden entstehen, das durch Knochengewebe gefüllt werden kann.

Eine durchgängige Porosität soll auch nach dem in J. Biomed. Mater. Res., Vol. 11, 1977, John Wiley & Sons, Inc., A. M. Rijke et al. « Porous Acrylic Cement », Seiten 373-394 beschriebenen Verfahren erzielt werden, bei dem ein löslicher Füllstoff dem Zement beigemischt wird. Als Füllstoffe werden darin Sucrose und Tricalciumphosphat beschrieben, die in Mengen bis zu 60 Gew.% und in einer Teilchengröße zwischen 53 und 297 µm dem Zement zugesetzt werden. Dabei wird am Modell eines mit Sucrose gefüllten Knochenzements festgestellt, daß eine durchgängige Porosität im Zement, in die ggf. Knochengewebe einwachsen kann, erst bei Sucrosegehalten oberhalb 40 Gew.% erreicht wird. Es wird in der Publikation darauf hingewiesen, daß Knochenzemente, worin eine solche Porosität erzeugt wurde, in ihrer Stabilität erheblich geschwächt werden, und es wird vorgeschlagen, die sehr schnell aus dem Knochenzement herausgelöste Sucrose durch Tricalciumphosphat zu ersetzen, so daß Herauslösen des Füllstoffs und Einwachsen von Knochen Hand in Hand geht und damit die Stabilität gewahrt bleibt.

Der Autor setzt dabei darauf, daß sich Tricalciumphosphat, zumindest auf lange Sicht, analog Sucrose aus dem Knochenzement herauslösen läßt. Über in vivo oder in vitro Versuche zur Bestätigung dieses Sachverhalts wird jedoch nicht berichtet. Tatsächlich findet man beim Einarbeiten von handelsüblichem Tricalciumphosphat in Knochenzement, daß damit keine Porosität in der gewünschten Dimension erzielt werden kann. Offensichtlich saugt sich beim Mischen des Tricalciumphosphats mit den für die Zementherstellung verwendeten Komponenten das poröse Tricalciumphosphat mit dem flüssigen Monomeren voll, was zur Folge hat, daß dies in den Tricalciumphosphatpartikeln auspolymerisiert und die Partikel somit weitgehend unlöslich werden. Das Ziel, eine zum Einwachsen von Knochen ausreichende Porosität zu erzielen, kann damit nicht erreicht werden.

In den Anmeldungen DE-A-2 620 890 und DE-A-2 620 907 werden zwar Tricalciumphosphate verwendet, die durch Sinterverfahren gewonnen wurden und die ein reduziertes Porenvolumen aufweisen. Aus der DE-A-2 620 907 ist zu entnehmen, daß die Restporosität noch 21 % ausmacht. In der DE-A-2 620 890 ist zwar kein Porenvolumen des gesinterten Materials angegeben, es muß jedoch angenommen werden, daß es in der gleichen Größe wie bei dem in DE-A-2 620 907 beschriebenen Material liegt. Abgesehen davon, daß beide Anmeldungen am gleichen Tag vom gleichen Anmelder mit im wesentlichen den gleichen Erfindern angemeldet sind und somit davon auszugehen ist, daß in beiden Fällen das als optimal erachtete gleiche Material verwendet wurde, kann auch aus dem Beispiel 2 der DE-A-2 620 890 geschlossen werden, daß das verwendete Material eine erhebliche Restporosität besessen hat. Dieses Beispiel ist das einzige der DE-A-2 620 890, worin Tricalciumphosphat (TCP) mit einem Knochenzement auf Acrylatbasis verarbeitet wird. Es wird darin eine Menge von 50 g TCP und 10 g Polymethylmethacrylat (Präpolymeres) mit 40 g Methylmethacrylat zu einer pastösen Masse angerührt, die dann aushärtet. Versuche zeigen, daß bei Verwendung von 60 g eines unporösen Feststoffes (Präpolymeres + TCP) etwa 22 bis 28 ml des flüssigen Monomeren benötigt wird, um zu einer gut modellierbaren pastösen Masse zu gelangen. Die Tatsache, daß in der DE-A-2 620 890 40 g, entsprechend 42,5 ml, Monomeres benötigt werden, zeigt, daß offenbar ein erheblicher Teil des Monomeren von dem TCP aufgesaugt wird, daß das verwendete TCP also ein erhebliches Restporenvolumen besaß. Auch bei diesem TCP ist also zu befürchten, daß durch in den Poren aushärtendes Acrylat die Resorption behindert wird. Schwerwiegender ist aber die Tatsache, daß durch die sehr stark erhöhte Menge des hochtoxischen Monomeren die Gefahr der Freisetzung von Monomeren in den Körper des Implantatträgers stark erhöht wird.

Es bestand daher die Aufgabe, einen Knochenzement mit Tricalciumphosphat zu finden, wobei durch Resorption des Tricalciumphosphats eine Porosität in der für das Einwachsen von Knochen vorteilhaften Dimension erzeugt wird, und wobei der Knochenzement sowohl unmittelbar nach dem Einbringen in den Körper schon eine ausreichende Stabilität aufweist, als auch in kurzer Zeit durch festes Verwachsen mit dem umgebenden Knochengewebe eine gute Langzeitstabilität garantiert.

Diese Aufgabe wurde durch die vorliegende Erfindung gelöst.

Es wurde nämlich gefunden, daß man durch Zumischung eines speziellen Tricalciumphosphats mit einem geringen verfügbaren Porenvolumen in einer bestimmten Menge und in einer genau definierten Teilchengröße zu den üblichen Knochenzementen auf Acrylatbasis einen Knochenzement erhält, der alle Forderungen bezüglich sowohl der Kurzzeit- als auch der Langzeitstabilität erfüllt.

Gegenstand der Erfindung ist demgemäß ein Knochenzement auf der Basis von aus Präpolymeren und Monomeren hergestellten Polyacrylaten oder Polymethacrylaten, der 5 bis 35 Gew.% resorbierbares Tricalciumphosphat mit einer Teilchengröße zwischen 50 und 300 μm enthält und der dadurch gekennzeichnet ist, daß das für die Monomere zugängliche Porenvolumen des Tricalciumphosphats weniger als 0,05 ml/g beträgt.

Als Tricalciumphosphate mit einem für Monomere zugänglichen Porenvolumen von weniger als 0,05 ml/g können sowohl gesinterte Tricalciumphosphate verwendet werden als auch handelsübliche gefällte Tricalciumphosphate mit einem höheren Porenvolumen als 0,05 ml/g, deren Porenvolumen vor dem Mischen mit dem Monomeren durch Auffüllen der Poren mit einem mit dem Monomeren nicht mischbaren Füllstoff auf den genannten Wert von weniger als 0,05 ml/g gebracht wurde.

Gegenstand der Erfindung ist auch die Verwendung von resorbierbarem Tricalciumphosphat mit einer Teilchengröße zwischen 50 und 300 μm in einer Menge von 5 bis 35 Gew.% als Zusatz zu Knochenzementen auf der Basis von aus Präpolymeren und Monomeren hergestellten Polyacrylaten oder Polymethacrylaten, dadurch gekennzeichnet, daß Tricalciumphosphat mit einem für die Monomere zugänglichen Porenvolumen von weniger als 0,05 ml/g verwendet wird.

Gegenstand der Erfindung ist ferner ein Verfahren zur Herstellung von Knochenzement auf der Basis von aus Präpolymeren und Monomeren hergestellten Polyacrylaten oder Polymethacrylaten durch Mischen von 5 bis 35 Gew.% resorbierbares Tricalciumphosphat mit einer Teilchengröße zwischen 50 und 300 μm mit einem Präpolymeren und einem die Polymerisation auslösenden Monomeren, dadurch gekennzeichnet, daß ein Tricalciumphosphat mit einem für die Monomere zugänglichen Porenvolumen von weniger als 0,05 ml/g verwendet wird und dieses, gegebenenfalls nach Vormischung mit dem Präpolymeren oder dem Monomeren, mit den übrigen Komponenten gemischt wird.

Die besonderen Vorteile der erfindungsgemäßen Knochenzemente liegen darin, daß durch den relativ geringen Anteil des Tricalciumphosphats der Verbund des zugrunde liegenden Acrylatpolymeren nicht negativ beeinflußt wird.

Im Gegensatz zu den Ergebnissen in dem oben zitierten J. Biomed. Mater. Res. wird sogar überraschenderweise eine Verbesserung der Druckbelastbarkeit des ausgehärteten Knochenzements beobachtet. Außerdem ist der normalerweise bei der Aushärtung von üblichen Knochenzementen zu beobachtende Volumenschwund deutlich verringert. Durch das zugesetzte Tricalciumphosphat wird auch die Wärmeableitung während des Polymerisationsprozesses verbessert, so daß die Gefahr von sogenannten Hitzenekrosen dadurch deutlich verringert ist.

Erstaunlicherweise wird bei dem erfindungsgemäßen Knochenzement ein sehr fester Verbund zwischen Implantat und Körpergewebe erreicht, obwohl es in der Regel nicht zu einer vollständigen Durchwachsung des Implantats durch Körpergewebe kommt, sondern nur die an der Oberfläche des Implantats liegenden Tricalciumphosphatpartikel resorbiert werden und die dadurch entstehende Randporosität durch Knochengewebe gefüllt wird. Durch die Teilchengröße des Tricalciumphosphats wird jedoch die Porengröße dieser Randporosität so gesteuert, daß Kanäle in für das Knochenwachstum besonders vorteilhaften Dimensionen entstehen. Nur dadurch wird eine primär knöcherne Auffüllung der Porositäten erreicht. Durch Resorption der Tricalciumphosphatpartikel in der Grenzzone kommt es zu einer nicht unerheblichen Vergrößerung der Kontaktfläche und dadurch zu einer Verteilung der auf die Grenzzone einwirkenden Kräfte auf eine größere Fläche.

Diese Kanäle sind, wie bereits erwähnt, in der Regel nur an der der Körperflüssigkeit zugängigen Oberfläche des Implantats. Wenn jedoch zufällig mehrere Tricalciumphosphatpartikel benachbart sind, reichen diese Kanäle auch weiter ins Innere des Implantats. Diese an der Oberfläche des Implantats vorhandene Porosität, die schnell durch Knochengewebe gefüllt werden kann, ist trotz der relativ geringen Menge von 5 bis 35 Gew.% an Tricalciumphosphat ausreichend, um den Verbund Im-

3

plantat/Knochen wesentlich zu verbessern.

Der Begriff Tricalciumphosphat, der in der vorliegenden Anmeldung gebraucht wird, its als Oberbegriff zu einer Anzahl von verschiedenen im wesentlichen durch die chemische Formel $Ca_3(PO_4)_2$ zu beschreibende Materialien zu verstehen, wobei das Verhältnis Ca zu P annähernd 3 : 2 beträgt. Neben den reinen Tricalciumphosphaten wie z. B. $\alpha$- oder $\beta$-Whitlockit, sollen jedoch auch die nur annähernd durch die Formel $Ca_3(PO_4)_2$ zu beschreibenden Materialien wie z. B. Apatite oder Phosphorit umfaßt sein. Auf jeden Fall soll das Tricalciumphosphat im Körper resorbierbar sein.

Diese Materialien sind an sich bekannt und können nach bekannten Verfahren hergestellt werden. Im wesentlichen sind dies Fällverfahren oder Sinterverfahren oder aber eine Kombination solcher Verfahren. Fäll- und Sinterverfahren zur Herstellung der Calciumphosphate sind in den Standardwerken der anorganischen Chemie, z. B. dem Gmelin, beschrieben. Als Ausgangsmaterialien dienen dazu in der Regel lösliche Calciumsalze und lösliche Phosphate oder aber für die Sinterverfahren z. B. CaO, CaOH, $CaCO_3$ und $CaHPO_4$, die mit $P_2O_5$ oder aber untereinander zusammengesintert werden.

Die bei den Fällverfahren gewonnenen Calciumphosphate sind in der Regel relativ weich und weisen ein großes Porenvolumen auf, das in der Größenordnung von etwa 0,3-0,5 ml/g liegt. Diese Calciumphosphate haben sowohl Vor- als auch Nachteile. Vorteilhaft ist, daß solche relativ porösen Calciumphosphate im Körper schnell resorbiert werden und ein schnelles Einwachsen von Knochengewebe in die dadurch gebildeten Porositäten des Zementes gestatten.

Nachteilig dabei ist jedoch, daß bei der Verarbeitung besondere Maßnahmen ergriffen werden müssen, um zu verhindern, daß sich die Calciumphosphatteilchen mit monomerem Acrylat bzw. Methacrylat vollsaugen. Dies ist unerwünscht, da hierdurch die schnelle, vollständige Polymerisation des Monomeren nicht mehr gewährleistet ist und die Gefahr besteht, daß ein höherer Anteil an Restmonomer nach dem Aushärten zurückbleibt und in den Kreislauf des Patienten gelangen kann. Außerdem wird durch das in den porösen Calciumphosphatteilchen polymerisierende Monomere die Resorbierbarkeit des Calciumphosphat herabgesetzt und es kann dadurch nicht zur Ausbildung von Poren in den gewünschten Dimensionen kommen.

Diese negativen Eigenschaften der üblichen, porösen Tricalciumphosphate, die eine Resorption weitgehend verhindern können, waren im Stand der Technik und insbesondere in der oben zitierten Publikation im J. Biomed. Mater. Res. nicht erkannt worden, da in dieser Arbeit Implantationsversuche und Versuche, bei denen der Füllstoff aus dem Zement herausgelöst wird, lediglich mit den sehr leicht löslichen und insbesondere unporösen Sucrosekristallen durchgeführt wurden, während mit Tricalciumphosphat lediglich Versuche zur Monomerfreisetzung und der Reduzierung der Polymerisationstemperatur durchgeführt wurden.

Die Aufnahme von flüssigem Monomeren in das poröse Calciumphosphat kann nämlich verhindert werden, wenn das Porenvolumen des Calciumphosphat vor dem Mischen mit dem Monomeren mit einem physiologisch verträglichen, vom Körper resorbierbaren und mit dem Monomeren nicht mischbaren Füllstoff gefüllt wird. Dadurch wird eine Aufnahme von Monomeren in das poröse Calciumphosphat verhindert. Als solche Füllstoffe geeignet sind beispielsweise Glycerin, Wasser bzw. wässerige Salz- oder Pufferlösungen, Äthylenglycol, niedermolekulare Polyäthylenglycole und niedere Alkohole wie Äthanol, n-Propanol und Isopropanol.

Um diese Komplikationen, hervorgerufen durch die poröse Struktur des verwendeten Calciumphosphats, auszuschließen, kann jedoch auch ein Calciumphosphat verwendet werden, das eine sehr geringe Porosität aufweist. Das Porenvolumen dieser Materialien muß unter 0,05 ml/g liegen. Man erhält diese Materialien in der Regel durch Sinterverfahren bei Temperaturen um oder über 1 000 °C bis etwa 1 500 °C. Als Ausgangsmaterialien können dazu neben den bereits oben erwähnten CaO, Ca(OH)₂, $CaCO_3$, $CaHPO_4$ und $P_2O_5$ auch gefällte poröse Tricalciumphosphate verwendet werden. Diese ursprünglich relativ weichen, porösen Materialien gewinnen schon durch ein etwa einstündiges Erhitzen auf etwa 1 200 °C, vorzugsweise nach vorherigem Verpressen, deutlich an Härte. Gleichzeitig geht dabei das Porenvolumen ganz drastisch zurück auf Werte, die deutlich unterhalb 0,05 ml/g liegen.

Die gleichen vorteilhaften Eigenschaften bezüglich der Härte und des Porenvolumens erhält man aber auch beim Sintern anderer Ausgangsmaterialien. So erhält man beispielsweise auch beim Zusammensintern eines Gemisches aus Calciumhydrogenphosphat und Calciumcarbonat bei etwa 1 200-1 500 °C ein Material mit einem Porenvolumen, das fast bei 0 liegt. Die Aufnahme von monomerem Acrylat bei der Verarbeitung ist bei diesen Materialien nicht zu befürchten, so daß hier auf eine Vorbehandlung des Calciumphosphats mit den obengenannten Füllmitteln verzichtet werden kann.

Die Gewinnung der Tricalciumphosphate in der definierten Teilchengröße geschieht durch an sich bekannte Methoden, z. B. durch Zermahlen und Sieben. Die Teilchengrößenfraktion von 50-300 μm kann aber auch z. B. durch Windsichten oder Sedimentieren gewonnen werden. Bevorzugt sind insbesondere Teilchen in der Größe von 80-200 μm.

Diese Tricalciumphosphatteilchen werden in Mengen zwischen 5 und 35 Gew.% vorzugsweise zwischen 15 und 30 Gew.% in den Knochenzement eingearbeitet. Die bekannten Knochenzemente werden so zubereitet, daß etwa 2 Teile eines feinteiligen, einen Polymerisationskatalysator (z. B. Dibenzoylperoxid) enthaltenden Präpolymerisats, insbesondere Polymethylmethacrylat oder eines Mischpolymerisats aus Methylacrylat und Methylmethacrylat, mit etwa einem Teil des flüssigen Monomeren, z. B. Acrylsäure- oder Methylacrylsäuremethylester oder deren Gemische, das einen Beschleuniger (z. B. Dimethyl-p-toluidin) enthält, zu einer formbaren Masse gemischt werden, die in den Körper implantiert wird und dort aushärtet. Solche Knochenzemente sind z. B. unter dem Warenzeichen Palacos® im Handel.

Die Herstellung der erfindungsgemäßen Knochenzemente erfolgt im Prinzip in ähnlicher Weise. Z. B. können die getrennten Komponenten, nämlich das den Polymerisationskatalysator enthaltende Präpolymerisat, das ggf. mit einem Füllstoff vorbehandelte Tricalciumphosphat und das den Beschleuniger enthaltende Monomere zusammengemischt werden. Es kann aber auch eine Vormischung von zweien der drei Komponenten in der Weise erfolgen, daß das Tricalciumphosphat entweder in Mischung mit dem Präpolymeren oder dem Monomeren vorliegt und diese Mischung dann mit der dritten Komponente vereint und gleichmäßig gemischt wird. Eine Vermischung des Tricalciumphosphat mit dem Präpolymeren und anschließendes Zumischen des Monomeren ist auch schon in dem oben zitierten J. Biomed. Mater. Res. beschrieben.

Die nach dem Stand der Technik verwendeten Präpolymeren sind ausnahmslos sogenannte Perlpolymerisate, die eine sehr glatte, einheitliche Oberfläche aufweisen. Da beim Aushärten der Zementmischung keine Kreuzreaktion zwischen dem Monomeren und dem Präpolymeren stattfindet, werden die Präpolymerkügelchen von dem sich neu bildenden Polymeren lediglich umschlossen, nicht jedoch daran gebunden. Dies kann dazu führen, daß insbesondere in den Randzonen des Implantats liegende, nicht allseitig umschlossene Präpolymerkügelchen relativ leicht aus dem Implantat herausgelöst und in den Körper abgeschwemmt werden, wo sie sich z. B. in Lymphknoten sammeln können.

Obwohl die erfindungsgemäßen Knochenzemente auch mit diesen konventionellen Perlpolymerisaten sehr vorteilhafte Ergebnisse liefern und diese Materialien deshalb von der vorliegenden Erfindung umfaßt sein sollen, werden vorzugsweise nicht kugelförmige Präpolymere verwendet, sondern Präpolymere, die aufgrund einer « eckigeren » Form sich nicht so leicht aus dem späteren Verbund herauslösen lassen. Bevorzugt werden deshalb Präpolymere, die in unregelmäßiger Granulatform oder als Schuppen (plattgedrückte Kugeln) oder als fadenförmige Zylinder vorliegen. Die Präpolymeren sollten einen Durchmesser von 10-80 μm, vorzugsweise einen solchen von 40-60 μm, besitzen. Die fadenförmigen Zylinder sollten eine Länge von 1-2 mm nicht überschreiten.

Falls ein poröses Tricalciumphosphat verwendet wird und deshalb eine Vorbehandlung des Tricalciumphosphats mit einem Füllmittel nötig ist, kann dieses Auffüllen der Poren auf verschiedene Weise erfolgen. In jedem Fall sollte diese Auffüllung der Poren so erfolgen, daß zwar alle Poren gefüllt sind, aber nicht so viel überschüssiges Füllmittel vorhanden ist, daß dieses die Tricalciumphosphatteilchen verklebt oder später das Aushärten der Implantationsmaterialien negativ beeinflußt.

Bei relativ niedrig viskosen Füllmitteln kann z. B. einfach die berechnete oder experimentell ermittelte Menge dem Tricalciumphosphat zugegeben werden, wobei auf Grund der Kapillarkräfte das Füllmittel in die Poren aufgesaugt wird. Bei höher viskosen Füllmitteln kann dieses jedoch auch in einem niedrig siedenden Lösungsmittel gelöst dem Tricalciumphosphat zugegeben werden und das Lösungsmittel dann verdampft werden. Vor allem bei relativ leicht flüchtigen Füllmitteln besteht jedoch auch die Möglichkeit der Porenfüllung über die Dampfphase. Dazu genügt es, das Tricalciumphosphat z. B. in einem geschlossenen System mit den sich über dem flüssigen Füllmittel bildenden Dämpfen in Berührung zu bringen, wobei auf Grund des in den Poren durch die Kapillarkräfte erniedrigten Dampfdrucks eine Kondensation des Füllmittels in den Poren bis zu deren vollständiger Füllung erfolgt.

Welche Methode jeweils am vorteilhaftesten ist, hängt von dem jeweils verwendeten Füllmittel ab. Bei der Verwendung von Glycerin als Füllmittel hat es sich beispielsweise als vorteilhaft erwiesen, das Glycerin in Alkohol gelöst dem Tricalciumphosphat zuzugeben und dann den Alkohol abdunsten zu lassen. Die Mengen, in denen die Füllmittel angewendet werden müssen, hängen ausschließlich von der Porosität des verwendeten Tricalciumphosphats ab, da, wie bereits gesagt, die Poren möglichst vollständig gefüllt sein sollen. Vorzugsweise wird jedoch ein Calciumphosphat verwendet, das so wenig porös ist, daß sich das Auffüllen der Poren erübrigt.

Zur Vermeidung von Infektionen des Implantatlagers, die auch durch sorgfältig aseptisches Arbeiten nicht immer verhindert werden können, kann dem erfindungsgemäßen Knochenzement auch ein Antibiotikum beigemischt werden. Insbesondere ist eine Beimischung von Gentamycin bevorzugt, mit dem sehr gute Ergebnisse erzielt werden. Die Verwendung von Gentamycin in Knochenzementen ist an sich u. a. durch die DE-A-2 022 117 bekannt. Trotzdem war es nicht naheliegend, dem erfindungsgemäßen, calciumphosphathaltigen Knochenzement Gentamycin beizumischen. Aus der Antibiotika-Fibel, 4. Auflage (1975), S. 373 unten bis 374 oben war nämlich bekannt, daß Gentamycin durch Calciumsalze, wie z. B. das Phosphat, in seiner Wirkung reduziert wird.

Überraschenderweise wurde jedoch gefunden, daß in die erfindungsgemäßen calciumphosphathaltigen Knochenzemente sogar weniger Gentamycin eingearbeitet werden muß als in die bekannten Knochenzemente ohne Calciumphosphat, um einen gleich guten Schutz gegen Infektionen des Implantatlagers zu erreichen. Obwohl die Menge des zugesetzten Antibiotikums in weiten Bereichen variiert werden kann und damit ganz dem beabsichtigten Effekt angepaßt werden kann, liegen die in die erfindungsgemäßen Materialien einzuarbeitenden Mengen an Gentamycin zur Erzielung einer bestimmten Wirkung durchweg niedriger als die für bekannte Knochenzemente notwendigen Mengen.

Für Gentamycin haben sich bei bekannten Knochenzementen Zusätze von etwa 1-4 Gew.% als besonders günstig erwiesen. Da die Freisetzungsrate von Gentamycin aus dem erfindungsgemäßen Knochenzement überraschenderweise bis zu etwa zehnmal höher ist als aus den bekannten gentamycinhaltigen Knochenzementen liegt dieser besonders bevorzugte Bereich bei den erfindungsgemäßen Knochenzementen bei 0,1-2 Gew.%. Geringere und auch höhere Zusätze von 0,02-4 Gew.% können jedoch für spezielle Zwecke bevorzugt sein.

Falls dem erfindungsgemäßen Material ein Antibiotikum beigemischt werden soll, können auch weitere Stoffe, wie z. B. die aus den DE-A-2 651 441 und DE-A-2 727 535 bekannten Aminosäuren, zugesetzt werden, durch die eine besonders gleichmäßige Freisetzung des Antibiotikums erreicht wird. Anstelle von Gentamycin können natürlich auch andere Antibiotika eingesetzt werden. In Betracht kommen grundsätzlich alle Antibiotika, die durch die bei der Polymerisation auftretenden Temperaturen nicht geschädigt werden, gegenüber den verwendeten Kunststoffen stabil sind und die in der erwünschten Weise aus dem Kunststoff freigesetzt werden. Aus der Vielzahl der in Frage kommenden Antibiotika seien nur beispielsweise die folgenden genannt: Erythromycin, Lincomycin, Clindamycin, Novobiocin, Vancomycin, Fusidinsäure, Rifampicin, Polymycine, Neomycin, Kanamycin und Tobramycin.

Das Mischen der Komponenten kann an sich in jedem geeigneten Behältnis erfolgen, z. B. einer Schale oder einem Becher. Es sind dazu auch schon spezielle Mischeinrichtungen vorgeschlagen worden. Wegen der Anwesenheit des relativ schweren Calciumphosphats muß das Mischen besonders sorgfältig erfolgen, um eine homogene Verteilung des Calciumphosphats im Knochenzement zu erreichen. Dabei besteht im besonderen Maße die Gefahr, daß Luft mit in die Masse eingerührt wird, die insbesondere in Form von Luftbläschen dann auch in dem ausgehärteten Knochenzement zugegen ist und dessen Stabilität mindern kann. Insbesondere auch wegen der schlechten Benetzbarkeit der Calciumphosphatoberfläche durch das flüssige Monomere lagern sich an den Calciumphosphatteilchen bevorzugt Luftbläschen an, die den Kontakt Calciumphosphat/Zement verhindern und dadurch die Stabilität des ausgehärteten Zements beträchtlich mindern. Die Untersuchung der herkömmlichen Knochenzemente hat außerdem gezeigt, daß als Folge von Fließeffekten um die Polymerkügelchen ebenfalls Luftblasen entstehen, die in der Regel zwischen 50 und 200 μm messen. Dieser Volumenanteil an eingeschlossenen Luftblasen kann im fertigen Zement bis zu 15 Volumenprozent ausmachen.

Diese Einschlüsse von Luftbläschen, die sowol bei den erfindungsgemäßen Knochenzementen unter Zusatz von Calciumphosphat als auch bei den Zementen nach dem Stand der Technik zu beobachten sind, lassen sich überraschenderweise durch eine relativ einfach durchzuführende Verfahrensmaßnahme zumindest so weit vermindern, daß die Stabilität der Implantate beträchtlich erhöht wird. Es hat sich nämlich gezeigt, daß eine Komprimierung der Mischung aus Tricalciumphosphat, Präpolymeren und Monomerem, oder auch nur von Präpolymerem und Monomeren in einer bestimmten Phase der Polymerisation die Bildung von Lufteinschlüssen weitgehend verhindert. Diese Komprimierung erfolgt im ersten Abschnitt der Verarbeitungsphase der Knochenzemente und schließt sich unmittelbar dem Mischen von Präpolymerem und Monomerem, bzw. Tricalciumphosphat, Propolymerem und Monomerem an.

Knochenzemente, die so eingestellt sind, daß sie nach etwa 6-15 Minuten nach dem Mischen ausgehärtet sind und etwa bis 2. bis 8. Minute verarbeitbar sind, werden vorzugsweise in der Zeit zwischen der 1. und 4. Minute einem erhöhten Druck ausgesetzt.

Dies geschieht vorzugsweise so, daß man die Mischung aus Tricalciumphosphat, Präpolymerem und Monomerem in einer Kolbenspritze passender Größe komprimiert, die mit Entlüftungslöchern versehen ist, deren Weite zwar nicht das Durchtreten der viskosen Mischung erlaubt, wohl aber das von eingeschlossener Luft und wobei der Spritzenstempel nicht luftdicht gegen die Spritzenwand abgeschlossen ist. Die hierfür notwendigen Drucke können durch ein Innengewinde der Spritze bei mäßigem Kraftaufwand erreicht werden, so daß es z. B. auch weiblichen Hilfspersonen ohne weiteres möglich ist, die erfindungsgemäßen Drucke zwischen 10 und 45 bar, vorzugsweise zwischen 20 und 40 bar zu erzeugen. Der Druck wird für einige Zeit aufrechterhalten, vorzugsweise für etwa 1-2 Minuten. Danach kann das Implantationsmaterial nach Entfernen der als Enflüftungsloch dienenden engen Ausläßkanüle durch einen ausreichend weiten Auslaßkanal aus der Spritze herausgepreßt und in den Körper eingebracht werden.

Knochenzemente mit oder ohne Calciumphosphat, die unter Vorkomprimierung hergestellt sind, weisen einen sehr stark verringerten Anteil an Lufteinschlüssen auf, wobei diese wenigen Einschlüsse nur einen sehr geringen Durchmesser besitzen. Bei den Knochenzementen, die Calciumphosphat enthalten, ist auffällig, daß bei Proben, die unter Vorkomprimierung hergestellt sind, ein fester Verbund des Polymeren mit dem Calciumphosphat vorliegt, der nicht durch Lufteinschlüsse an der Calciumphosphatoberfläche gestört ist. Materialien, die unter Vorkomprimierung hergestellt sind, weisen daher eine erheblich größere Festigkeit auf als solche, die nach dem Mischen direkt in den Körper eingebracht sind. Die Verarbeitung der erfindungsgemäßen Knochenzemente unter Vorkomprimierung ist daher ein weiterer wesentlicher Aspekt der vorliegenden Erfindung.

Ein besonderer Vorteil der erfindungsgemäßen Knochenzemente ist auch die Tatsache, daß auf die Zumischung eines Röntgenkontrastmittels verzichtet werden kann. Als solches wird den üblichen Knochenzementen in der Regel Bariumsulfat zugemischt, das jedoch aufgrund seiner Toxizitat relativ große Probleme aufwirft. Das in den erfindungsgemäßen Knochenzementen enthaltene Calciumphosphat bietet jedoch eine ausreichend starke Röntgenabsorption, so daß kein zusätzliches Röntgenkontrastmittel beigemischt werden muß.

Die erfindungsgemäßen Knochenzemente, insbesondere solche, die nach dem erfindungsgemäßen

6

Verfahren auch unter Vorkomprimierung hergestellt sind, können sehr vorteilhaft bei der Bekämpfung von Knochendefekten verwendet werden. Sie dienen dabei einmal zur Implantation von Endoprothesen, zum anderen aber auch zur Durchführung von sogenannten Verbundosteosynthesen, und zur Auffüllung von bestimmten Knochendefekten.

Nachfolgend wird an Hand von Beispielen die Erfindung näher erläutert.

### Beispiel 1

40 g eines gefällten Tricalciumphosphats der chemischen Zusammensetzung $Ca_5(PO_4)_3OH$ mit einem Porenvolumen von 0,4 ml/g werden mit 20 ml Glycerin in 50 ml absolutem Alkohol versetzt und in einem Exsikkator 24 Stunden zur Entfernung des Alkohols stehengelassen. 5 dieses vorbehandelten Tricalciumphosphats, das eine mittlere Teilchengröße zwischen 100 und 200 μm aufweist, werden mit 5,5 ml Methacrylsäuremethylester, enthaltend 0,7 % Dimethyl-p-toluidin und etwa 0,006 % Hydrochinon (im folgenden « Monomer » genannt) und 10,0 g eines feinteiligen (Teilchendurchmesser 30 μm) Copolymerisats von Acryl- und Methacrylsäuremethylester, enthaltend 0,5 % Dibenzoxylperoxid und Spuren Chlorophyll (im folgenden « Präpolymer » genannt) gut gemischt.

Die Masse wird von der 1. bis zur 3. Minute nach dem Mischen unter einem Druck von etwa 35 bar komprimiert und ist innerhalb der ersten 3 bis 6 Minuten verarbeitbar, bis etwa zur 8. Minute noch plastisch verformbar und nach etwa 10-11 Minuten fest. Der erhaltene Zement ist gut hart und zeigt keine Reste von Glycerin and der Oberfläche. Ähnlich gute Resultate werden bei Verwendung von 7 ml und insbesondere bei Verwendung vom 6 ml Monomer erhalten.

### Beispiel 2

40 g des gefällten Tricalciumphosphats von Beispiel 1 werden mit 30 ml Glycerin in 40 ml absoluten Alkohol versetzt und 24 Stunden in einem Exsikkator zur Entfernung des Alkohols stehen gelassen. 5 g dieses so behandelten Tricalciumphosphats werden mit 6 ml Monomer und 10 g Präpolymer gut gemischt. Die Masse läßt sich sehr gut mischen und ist von der 2. bis zur 9. Minute spritzbar, bis zur 11. Minute von Hand knetbar, nach etwa 12 Minuten warm und nach etwa 13 Minuten erhärtet. Der erhaltende Zement ist sehr schön hart und die Oberfläche ist frei von Glycerinresten. Eine Komprimierung auf einen Druck von etwa 35 bar zwischen der 1. und 3. Minute.

### Beispiel 3a

Die folgenden Ausgangsmaterialien

A ein gefälltes Tricalciumphosphat mit einem Porenvolumen von 0,35 ml/g (E. Merck, Art. Nr. 2143)
B ein gefälltes Tricalciumphosphat mit einem Porenvolumen von 0,40 ml/g
C eine innige Mischung aus 2 Teilen Calciumhydrogenphosphat und einem Teil Calciumcarbonat

werden mit einer Exzenterpresse vorgranuliert und anschließend zu Tabletten verpreßt.

Die Ausgangsmaterialien A und B werden danach jeweils 1 Stunde bei 600°, 900°, 1 200° und 1 500 °C geglüht und Ausgangsmaterial C zunächst 3 Stunden bei 1 200 °C geglüht, danach grob zerteilt, nochmals zu Tabletten verpreßt und für eine Stunde bei 1 500 °C geglüht.

In der folgenden Tabelle sind die spezifischen Oberflächen und spezifischen Porenvolumina der so behandelten Ausgangsmaterialen dargestellt.

| Material | Temperatur (°C) | Spez. Oberfläche (cm²/g) | Spez. Porenvolumen (ml/g) |
|---|---|---|---|
| A | | 50,8 | 0,35 |
| A | 600 °C | 27,8 | 0,30 |
| A | 900 °C | 10,4 | 0,29 |
| A | 1 200 °C | 3,4 | 0,02 |
| A | 1 500 °C | 0,8 | 0,01 |
| B | | 73,2 | 0,40 |
| B | 600 °C | 20,9 | 0,30 |
| B | 900 °C | 6,0 | 0,04 |
| B | 1 200 °C | 2,0 | 0,01 |
| B | 1 500 °C | 0,6 | 0,01 |
| C | 1 200 °C | 2,1 | 0,01 |
| C | 1 500 °C | 1,1 | 0,01 |

Eine kristallographische Untersuchung der Materialien zeigt, daß A und B bei allen Glühtemperaturen eine Hydroxylapatit-Struktur besitzen, während C eine Whitlockit-Struktur besitzt.

Die geglühten Materialien können durch Zerkleinern und Klassieren in allen gewünschten Teilchengrößen gewonnen werden.

## Beispiel 3b

5 g des bei 1 200 °C geglühten Tricalciumphosphats A nach Biespiel 3a mit einer Teilchengröße zwischen 63 und 200 μm werden mit 6 ml Monomer und 10 g Präpolymer gut gemischt. Von etwa der 1. bis etwa zur 3. Minute wird die Mischung unter einem Druck von etwa 35 bar komprimiert. Die Masse ist bis zur 6. Minute spritzbar, bis zur 8. Minute von Hand knetbar und plastisch deformierbar, nach etwa 9 Minuten warm und nach etwa 10 Minuten erhärtet. Der erhaltene Knochenzement ist gut hart.

In gleicher Weise und mit gleich guten Ergebnissen können auch die bei 1 200-1 500 °C geglühten Materialien B und C verwendet werden.

## Beispiel 4

20 g des bei 1 200 °C geglühten Tricalciumphosphats A nach Beispiel 3a mit einer Teilchengröße zwischen 50 und 300 μm werden mit 24 ml Monomer und 40 g Präpolymer gut gemischt. Von etwa der 1. bis zur 3. Minute wird die Mischung unter einem Druck von etwa 35 bar in einer Kolbenspritze komprimiert.

Ein arthrotisch verändertes Hüftgelenk wird eröffnet, der Schenkelhals freipräpariert, die Kapsel entfernt und der Hüftknopf mit dem Schenkelhals 1 cm oberhalb des Trochanter minors in einem Winkel zur Horizontalebene von 35-45 Grad reseziert, die Markhöhle kurettiert und mit einer Spezialfräse aufbereitet, so daß eine künstliche Metallprothese eingepaßt werden kann. In die so vorbereitete Femurmarkhöhle wird ein Entlüftungsschlauch eingeführt, der bis unterhalb die Prothesenspitze reicht.

In die ausgespülte und in dieser Weise präparierte Markhöhle wird nun die in der oben beschriebenen Weise zubereitete Knochenzementmasse injiziert und anschließend die Metallschaftsprothese so eingeführt, daß der Metallschaft vollständig von Knochenzement umgeben ist und diesen Kunststoffköcher nirgends perforiert. Dies wird dadurch erreicht, daß Implantationswinkel und Resektionsflächen durch praeoperative Zeichnungen geplant sind, wodurch die Stelle des Eintauchens der Metallprothese in den plastischen Zement vorher bestimmt werden kann. In dieser Position wird die implantierte Metallprothese unverrückbar bis zur elften Minute unter Druck eingepreßt gehalten. Nach dieser Zeit ist der Knochenzement ausgehörtet und die Prothese stabil implantiert.

Die in der Grenzzone auftretenden Schrumpfungsspalten sind so klein, daß bei normaler Knochenvaskularisation der Spalt nach spätestens 12 Wochen knöchern aufgefüllt wird, so daß die künstliche Prothese von diesem Zeitpunkt an voll belastet werden kann.

## Beispiel 5

Ein arthrotischer, aber in seiner äußeren Form noch gut erhaltener Hüftkopf kann auch für eine sogenannte Kappenprothese vorbereitet werden. Hierfür wird der Hüftkopf von seinem Restknorpel befreit. Die Knorpelgrundplatte wird mit einer Spezialfräse aufbereitet, so daß blutende Knochenpunkte sichtbar werden. Die Hüftkappe wird in der Weise angepaßt, daß die Grenzzone Knochen und Zement in erster Linie auf Druckkräfte beansprucht wird, d. h. entgegen der physiologischen Winkelstellung des Hüftkopfes wird die Kappe valgisiert und deflektiert auf den Hüftkopf aufgebracht.

Anschließend wird der Hüftkopf getrocknet und die Kappenprothese mit einer 2 mm dicken Schicht eines Zementes belegt, der in der im Beispiel 4 beschriebenen Weise zubereitet worden ist. Die den Zement enthaltende Kappe wird nun über den gut trocken Hüftkopf gestülpt und fest angepreßt. Der dabei hervorquellende Zement wird entfernt und die Kappe bis zu elften Minute in der sorgfältig geplanten Position angepreßt gehalten. Die so fixierte Metallkappe zeigt sofort einen ausreichend stabilen Sitz, so daß der überkappte Femurkopf ohne Schwierigkeiten in die ins Becken implantierte Kunststoffpfanne reponiert werden kann.

## Beispiel 6

In Fällen von Knochenfrakturen, insbesondere von sogenannten pathologischen Frakturen bei Knochentumoren oder Knochenmetastasen anderer Primärtumoren kann eine Verbundosteosynthese zur Anwendung kommen. Hierbei werden zwei Fragmente, in denen Metallschrauben nicht mehr sicher fixiert werden können in der Weise mit einem Knochenzement, der nach Beispiel 4 zubereitet worden ist, vom Markraum her ausgefüllt, daß entweder direkt in den weichen Zement oder nach Bohren und Gewindeschneiden des ausgehärteten Zementes, auf diesen Verbund von Knochen und Knochenzement, eine Metallplatte mit Schrauben fixiert werden kann.

Eine solche Verbundosteosynthese kann auch ausgeführt werden, indem der Verbund von Zement und Knochen durch einen intramedullären Metallnagel verstärkt wird, der die Fragmente gleichsam auffädelt. Nach Aushärten des Zementes bekommen die Schrauben, bzw. die Metallimplantate einen festen Halt, so daß es zur schnellen Knochenheilung kommen kann. Es konnte gezeigt werden, daß die Knochenheilung durch die Zementplombierung nicht gestört wird.

## Beispiel 7

In gewissen Fällen, in denen epiphysennah Knochentumore aufgetreten sind, können große knöcherne Defekte, bei denen eine Auffüllung durch körpereigene Knochen nicht mehr zu etwarten ist und auf Grund derer eine Belastbarkeit der Extremität nicht mehr gewährleistet ist, mit Knochenzementen aufgefüllt und die Extremitäten mit oder ohne Metallarmierung mit dem nach Beispiel 4 aufbereiteten Knochenzement, aufgefüllt werden. Die so behandelten Knochen erreichen wieder eine Belastungsstabilität oder zumindest eine Übungsstabilität, die zu einer erheblichen Pflegeerleichterung beiträgt.

## Beispiele 8-10

Die Beispiele 1-3 werden wiederholt, wobei anstelle des dort verwendeten feinteiligen Präpolymeren ein Polymergranulat mit einer Teilchengröße von 40-60 μm verwendet wird.

## Beispiele 11-13

Die Beispiele 1-3 werden wiederholt, wobei anstelle des dort verwendeten feinteiligen Präpolymeren fadenförmige Polymerzylinder mit einer Länge von 0,5 bis 1 mm und einem Durchmesser von etwa 50 μm verwendet werden.

## Beispiel 14

Aus 30 g Präpolymer, 21 ml Monomer, 15 g Tricalciumphosphat und 0,5 g Gentamycin-Base wird eine Knochenzementmischung bereitet, aus der zylinderförmige Prüfkörper mit einem Durchmesser von 25 mm und einer Höhe von 10 mm geformt werden. Ein solcher Prüfkörper wird in 20 ml Phosphatpuffer von pH 7,4 eingebracht. Nach jeweils 24 Stunden wird die Flüssigkeit gewechselt und auf ihren Gentamycingehalt untersucht. Als Vergleich dient ein analoger Prüfkörper, der aus einer Mischung von 40 g Präpolymer, 20 ml Monomer und 0,5 g Gentamycinbase geformt wurde.

Der Vergleich brachte das folgende Ergebnis :

| | Gentamycin-Gehalt in der Flüssigkeit [μg/ml] | |
| | konventioneller Knochenzement | erfindungsgemäßer Knochenzement |
| --- | --- | --- |
| 1. Tag | 83 | 260 |
| 2. Tag | 12 | 119 |
| 3. Tag | 5,6 | 67 |
| 4. Tag | 4,5 | 52 |
| 5. Tag | 3,8 | 38 |

**Ansprüche** (für die Vertragsstaaten : BE, CH, DE, FR, GB, IT, NL, SE)

1. Knochenzement auf der Basis von aus Präpolymeren und Monomeren hergestellten Polyacrylaten oder Polymethacrylaten, der 5 bis 35 Gew.% resorbierbares Tricalciumphosphat mit einer Teilchengröße zwischen 50 und 300 μm enthält, dadurch gekennzeichnet, daß das für die Monomere zugängliche Porenvolumen des Tricalciumphosphats weniger als 0,05 ml/g beträgt.

2. Knochenzement nach Anspruch 1, dadurch gekennzeichnet, daß zusätzlich ein pharmazeutischer Wirkstoff enthalten ist.

3. Knochenzement nach Anspruch 2, dadurch gekennzeichnet, daß als Wirkstoff Gentamycin enthalten ist.

4. Knochenzement nach Anspruch 3, dadurch gekennzeichnet, daß das Gentamycin in einer Menge von 0,1 bis 2 Gew.% enthalten ist.

5. Verwendung von resorbierbarem Tricalciumphosphat mit einer Teilchengröße zwischen 50 und 300 μm in einer Menge von 5 bis 35 Gew.% als Zusatz zu Knochenzement auf der Basis von aus Präpolymeren und Monomeren hergestellten Polyacrylaten oder Polymethacrylaten, dadurch gekennzeichnet, daß Tricalciumphosphat mit einem für die Monomere zugänglichen Porenvolumen von weniger als 0,05 ml/g verwendet wird.

6. Verfahren zur Herstellung von Knochenzement auf der Basis von aus Präpolymeren und Monomeren hergestellten Polyacrylaten oder Polymethacrylaten durch Mischen von 5 bis 35 Gew.% resorbierbares Tricalciumphosphat mit einer Teilchengröße zwischen 50 und 300 μm mit einem Präpolymeren und einem die Polymerisation auslösenden Monomeren, dadurch gekennzeichnet, daß ein Tricalciumphosphat mit einem für die Monomere zugänglichen Porenvolumen von weniger als 0,05 ml/g verwendet wird und dieses, gegebenenfalls nach Vormischung mit dem Präpolymeren oder dem Monomeren, mit den übrigen Komponenten gemischt wird.

7. Verfahren nach Anspruch 6, dadurch gekennzeichnet, daß ein poröses Tricalciumphosphat verwendet wird, dessen Porenvolumen vor der Versmichung mit den übrigen Komponenten mit einem Körperverträglichen, resorbierbaren und mit dem Monomeren nicht mischbaren Füllstoff gefüllt wird.

8. Verfahren nach Anspruch 6, dadurch gekennzeichnet, daß als Tricalciumphosphat mit einem für die Monomere zugänglichen Porenvolumen von weniger als 0,05 ml/g gesintertes Tricalciumphosphat verwendet wird.

9. Verfahren nach einem der Ansprüche 6 bis 8, dadurch gekennzeichnet, daß die Mischung vor dem Aushärten unter einem Druck von 10-45 bar komprimiert wird.

10. Verfahren nach einem der Ansprüche 6 bis 9, dadurch gekennzeichnet, daß das Präpolymere einen Teilchendurchmesser von 10 bis 80 $\mu$m besitzt.

11. Verfahren nach einem der Ansprüche 6 bis 10, dadurch gekennzeichnet, daß das Präpolymere in Granulat- oder Schuppenform oder als fadenförmige Zylinder mit einer Länge von 0,1-2 mm vorliegt.

12. Verfahren nach einem der Ansprüche 6 bis 11, dadurch gekennzeichnet, daß zusätzlich ein Antibiotikum eingearbeitet wird.

**Ansprüche** (für den Vertragsstaat AT)

1. Verfahren zur Herstellung von Knochenzement auf der Basis von aus Präpolymeren und Monomeren hergestellten Polyacrylaten oder Polymethacrylaten durch Mischen von 5 bis 35 Gew.% resorbierbares Tricalciumphosphat mit einer Teilchengröße zwischen 50 und 300 $\mu$m mit einem Präpolymeren und einem die Polymerisation auslösenden Monomeren, dadurch gekennzeichnet, daß ein Tricalciumphosphat mit einem für die Monomere zugänglichen Porenvolumen von weniger als 0,05 ml/g verwendet wird und dieses, gegebenenfalls nach Vormischung mit dem Präpolymeren oder dem Monomeren, mit den übrigen Komponenten gemischt wird.

2. Verfahren nach Anspruch 1, dadurch gekennzeichnet, daß ein poröses Tricalciumphosphat verwendet wird, dessen Porenvolumen vor der Vermischung mit den übrigen Komponenten mit einem körperverträglichen, resorbierbaren und mit dem Monomeren nicht mischbaren Füllstoff gefüllt wird.

3. Verfahren nach Anspruch 1, dadurch gekennzeichnet, daß als Tricalciumphosphat mit einem für die Monomere zugänglichen Porenvolumen von weniger als 0,05 ml/g gesintertes Tricalciumphosphat verwendet wird.

4. Verfahren nach einem der Ansprüche 1 bis 3, dadurch gekennzeichnet, daß die Mischung vor dem Aushärten unter einem Druck von 10-45 bar komprimiert wird.

5. Verfahren nach einem der Ansprüche 1 bis 4, dadurch gekennzeichnet, daß das Präpolymere einen Teilchendurchmesser von 10 bis 80 $\mu$m besitzt.

6. Verfahren nach einem der Ansprüche 1 bis 5, dadurch gekennzeichnet, daß das Präpolymere in Granulat- oder Schuppenform oder als fadenförmige Zylinder mit einer Länge von 0,1-2 mm vorliegt.

7. Verfahren nach einem der Ansprüche 1 bis 6, dadurch gekennzeichnet, daß zusätzlich ein Antibiotikum eingearbeitet wird.

8. Verfahren nach Anspruch 7, dadurch gekennzeichnet, daß Gentamycin eingearbeitet wird.

9. Verfahren nach Anspruch 8, dadurch gekennzeichnet, daß das Gentamycin in einer Menge von 0,1 bis 2 Gew.% eingearbeitet wird.

10. Verwendung von resorbierbarem Tricalciumphosphat mit einer Teilchengröße zwischen 50 und 300 $\mu$m in einer Menge von 5 bis 35 Gew.% als Zusatz zu Knochenzement auf der Basis von aus Präpolymeren und Monomeren hergestellten Polyacrylaten oder Polymethacrylaten, dadurch gekennzeichnet, daß Tricalciumphosphat mit einem für die Monomere zugänglichen Porenvolumen von weniger als 0,05 ml/g verwendet wird.

**Claims** (for the Contracting States : BE, CH, DE, FR, GB, IT, NL, SE)

1. Bone cement on the basis of polyacrylates or polymethacrylates produced from prepolymers and monomers, containing 5 to 35 % by weight of resorbable tricalcium phosphate of a particle size between 50 and 300 $\mu$m, characterized in that the pore volume of the tricalcium phosphate which is accessible to the monomer is less than 0.05 ml/g.

2. Bone cement according to claim 1, characterized in that it contains additionally a pharmaceutically active agent.

3. Bone cement according to claim 2, characterized in that Gentamycin is contained as active agent.

4. Bone cement according to claim 3, characterized in that Gentamycin is contained in an amount of 0.1 to 2 % by weight. ˙

5. Use of a resorbable tricalcium phosphate of a particle size between 50 and 300 $\mu$m as an additiv to bone cement on the basis of polyacrylates or polymethacrylates produced from prepolymers and monomers, characterized in that tricalcium phosphate with a pore volume accessible to the monomer of less than 0.05 ml/g is used.

6. Process for the production of bone cement on the basis of polyacrylates or poylmethacrylates

0 016 906

produced from prepolymers and monomers by mixing of 5 to 35 % by weight of a resorbable tricalcium phosphate of a particle size between 50 and 300 μm with a prepolymer and a monomer which starts the polymerization, characterized in that a tricalcium phosphate with a pore volume accessible to the monomer of less than 0.05 ml/g is used, and is, possibly after premixing with the prepolymer or the monomer, mixed with the remaining components.

7. Process according to claim 6, characterized in that a porous tricalcium phosphate is used the pore volume of which is filled with a filler which is physiologically compatible, resorbable and non-miscible with the monomer, prior to the mixing with the remaining components.

8. Process according to claim 6, characterized in that as the tricalcium phosphate with a pore volume accessible to the monomer of less than 0.05 ml/g, a sintered tricalcium phosphate is used.

9. Process according to one of claims 6 to 8, characterized in that the mixture is compressed under a pressure of from 10 to 45 bar, prior to hardening.

10. Process according to one of claims 6 to 9, characterized in that the prepolymer has a particle diameter of from 10 to 80 μm.

11. Process according to one of claims 6 to 10, characterized in that the prepolymer is in the form of granules, flakes or thread-like cylinders of a length of from 0.1 to 2 mm.

12. Process according to one of claims 6 to 11, characterized in that additionally an antibiotic is worked in.

Claims (for the Contracting State AT)

1. Process for the production of bone cement on the basis of polyacrylates or poylmethacrylates produced from prepolymers and monomers by mixing of 5 to 35 % by weight of a resorbable tricalcium phosphate of a particle size between 50 and 300 μm with a prepolymer and a monomer which starts the polymerization, characterized in that a tricalcium phosphate with a pore volume accessible to the monomer of less than 0.05 ml/g is used, and is, possibly after premixing with the prepolymer or the monomer, mixed with the remaining components.

2. Process according to claim 1, characterized in that a porous tricalcium phosphate is used the pore volume in which is filled with a filler which is physiologically compatible, resorbable and non-miscible with the monomer, prior to the mixing with the remaining components.

3. Process according to claim 1, characterized in that as the tricalcium phosphate with a pore volume accessible to the monomer of less than 0.05 ml/g, a sintered tricalcium phosphate is used.

4. Process according to one of claims 1 to 3, characterized in that the mixture is compressed under a pressure of from 10 to 45 bar, prior to hardening.

5. Process according to one of claims 1 to 4, characterized in that the prepolymer has a particle diameter of from 10 to 80 μm.

6. Process according to one of claims 1 to 5, characterized in that the prepolymer is in the form of granules, flakes or thread-like cylinders of a length of from 0.1 to 2 mm.

7. Process according to one of claims 1 to 6, characterized in that additionally and antibiotic is worked in.

8. Process according to claim 7, characterized in that Gentamycin is worked in.

9. Process according to claim 8, characterized in that the Gentamycin is worked in in an amount of from 0.1 to 2 % by weight.

10. Use of a resorbable tricalcium phosphate of a particle size between 50 and 300 μm as an additiv to bone cement on the basis of polyacrylates or polymethacrylates produced from prepolymers and monomers, characterized in that tricalcium phosphate with a pore volume accessible to the monomer of less than 0.05 ml/g is used.

Revendications (pour les Etats contractants : BE, CH, DE, FR, GB, IT, NL, SE)

1. Ciment osseux à base de polyacrylates ou de polyméthacrylates préparés à partir de prépolymères et de monomères, ce ciment contenant 5 à 35 % en poids de phosphate tricalcique résorbable d'une granularité se situant entre 50 et 300 μm, caractérisé en ce que le volume de pores du phosphate tricalcique, qui est accessible pour les monomères, est inférieur à 0,05 ml/g.

2. Ciment osseux suivant la revendication 1, caractérisé en ce qu'il contient, en outre, une substance pharmaceutique active.

3. Ciment osseux suivant la revendication 2, caractérisé en ce que la gentamycine y est contenue comme substance active.

4. Ciment osseux suivant la revendication 3, caractérisé en ce que la gentamycine y est contenue en une quantité de 0,1 à 2 % en poids.

5. Utilisation de phosphate tricalcique résorbable d'une granularité se situant entre 50 et 300 μm, en une quantité de 5 à 35 % en poids, comme additif à un ciment osseux à base de polyacrylates ou de polyméthacrylates préparés à partir de prépolymères et de monomères, caractérisée en ce qu'on utilise

11

du phosphate tricalcique ayant un volume de pores accessible pour les monomères de moins de 0,05 ml/g.

6. Procédé de préparation d'un ciment osseux à base de polyacrylates ou de polyméthacrylates préparés à partir de prépolymères et de monomères par mélange de 5 à 35 % en poids de phosphate tricalcique résorbable d'une granularité se situant entre 50 et 300 μm avec un prépolymère et un monomère déclenchant la polymérisation, caractérisé en ce qu'on utilise un phosphate tricalcique ayant un volume de pores accessible pour les monomères de moins de 0,05 ml/g et on mélange ce phosphate tricalcique avec les autres composants éventuellement après mélange préalable avec le prépolymère ou le monomère.

7. Procédé suivant la revendication 6, caractérisé en ce qu'on utilise un phosphate tricalcique poreux dont le volume de pores est rempli, avant le mélange avec les autres composants, d'une charge compatible avec le corps, résorbable et non miscible avec le monomère.

8. Procédé suivant la revendication 6, caractérisé en ce que, comme phosphate tricalcique d'un volume de pores accessible pour les monomères de moins de 0,05 ml/g on utilise un phosphate tricalcique fritté.

9. Procédé suivant une des revendications 6 à 8, caractérisé en ce que, avant le durcissement, on comprime le mélange sous une pression de 10-45 bars.

10. Procédé suivant une des revendications 6 à 9, caractérisé en ce que le prépolymère est en particules d'un diamètre de 10 à 80 μm.

11. Procédé suivant une des revendications 6 à 10, caractérisé en ce que le prépolymère est sous forme de granules, de paillettes ou de cylindres filiformes d'une longueur de 0,1 à 2 mm.

12. Procédé suivant une des revendications 6 à 11, caractérisé en ce qu'on incorpore, en outre, un antibiotique.


**Revendications** (pour l'Etat contractant AT)

1. Procédé de préparation d'un ciment osseux à base de polyacrylates ou de polyméthacrylates préparés à partir de prépolymères et de monomères en mélangeant 5 à 35 % en poids de phosphate tricalcique résorbable d'une granularité se situant entre 50 et 300 μm avec un prépolymère et un monomère déclenchant la polymérisation, caractérisé en ce qu'on utilise un phosphate tricalcique ayant un volume de pores accessible pour les monomères de moins de 0,05 ml/g et on mélange ce phosphate tricalcique avec les autres composants éventuellement après mélange préalable avec le prépolymère ou le monomère.

2. Procédé suivant la revendication 1, caractérisé en ce qu'on utilise un phosphate tricalcique poreux dont le volume de pores est rempli, avant le mélange avec les autres composants, d'une charge compatible avec le corps, résorbable et non miscible avec le monomère.

3. Procédé suivant la revendication 1, caractérisé en ce que, comme phosphate tricalcique d'un volume de pores accessibles pour les monomères de moins de 0,05 ml/g on utilise un phosphate tricalcique fritté.

4. Procédé suivant une des revendications 1 à 3, caractérisé en ce que, avant le durcissement, on comprime le mélange sous une pression de 10-45 bars.

5. Procédé suivant une des revendications 1 à 4, caractérisé en ce que le prépolymère est en particules d'un diamètre de 10 à 80 μm.

6. Procédé suivant une des revendications 1 à 5, caractérisé en ce que le prépolymère est sous forme de granules, de paillettes ou de cylindres filiformes d'une longueur de 0,1 à 2 mm.

7. Procédé suivant une des revendications 1 à 6, caractérisé en ce qu'on incorpore, en outre, un antibiotique.

8. Procédé suivant la revendication 7, caractérisé en ce qu'on incorpore la gentamycine.

9. Procédé suivant la revendication 8, caractérisé en ce qu'on incorpore la gentamycine en une quantité de 0,1 à 2 % en poids.

10. Utilisation de phosphate tricalcique résorbable d'une granularité se situant entre 50 et 300 μm, en une quantité de 5 à 35 % en poids, comme additif à un ciment osseux à base de polyacrylates ou de polyméthacrylates préparés à partir de prépolymères et de monomères, caractérisé en ce qu'on utilise du phosphate tricalcique avant un volume de pores accessible pour les monomères de moins de 0,05 ml/g.